(19) Europäisches Patentamt
European Patent Office
Office européen des brevets

(11) Numéro de publication: **0 258 160**
**A2**

(12) # DEMANDE DE BREVET EUROPEEN

(21) Numéro de dépôt: **87420220.3**

(22) Date de dépôt: **12.08.87**

(51) Int. Cl.[4]: **C 07 D 307/28**
C 07 D 405/06
// C07C43/178

(30) Priorité: **22.08.86 FR 8612097**

(43) Date de publication de la demande:
**02.03.88 Bulletin 88/09**

(84) Etats contractants désignés:
**AT BE CH DE ES FR GB GR IT LI LU NL SE**

(71) Demandeur: **RHONE-POULENC AGROCHIMIE**
**14-20, rue Pierre Baizet**
**F-69009 Lyon (FR)**

(72) Inventeur: **Corbet, Jean Pierre**
**7 Chemin de Charrière Blanche**
**F-69130 Ecully (FR)**

**Mas, Jean Manuel**
**1 rue du Tonkin**
**F-69100 Villeurbanne (FR)**

(74) Mandataire: **Ranguis, Patrick Frédéric et al**
**RHONE POULENC AGROCHIMIE Service DPI B.P. 9163**
**F-69263 Lyon Cédex 09 (FR)**

(54) **Dérivés de 2,3 dihydrofuranne leur procédé de préparation, leur utilisation comme intermédiaire pour la préparation de tétrahydrofuranne.**

(57) L'invention concerne des dérivés de 2,3-dihydrofuranne de formule :

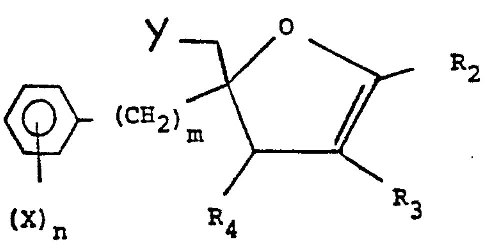

(I)

- X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano dans le cas où $R_3$ et/ou $R_4$ correspondent à l'atome d'hydrogène.
- n est un nombre entier, positif ou nul, inférieur à 6, de préférence égal à 2, étant entendu que lorsque n est supérieur à 1, les substituants X peuvent être soit identiques, soit différents,
- m = 0 ou 1,
- Y correspond à un atome ou groupement susceptible d'être éliminé au moyen d'une substitution nucléophile, éventuellement après transformation intermédiaire appropriée.

leurs sels acceptables en agriculture.

Utilisation de ces dérivés comme intermédiaires dans le procédé de préparation de tétrahydrofuranne à groupe triazole utiles comme fongicides.

EP 0 258 160 A2

**Description**

DERIVES DE 2,3 DIHYDROFURANNE, LEUR PROCEDE DE PREPARATION, LEUR UTILISATION COMME INTERMEDIAIRE POUR LA PREPARATION DE TETRAHYDROFURANNE

L'invention concerne des dérivés de 2,3-dihydrofuranne. Elle concerne également un procédé de préparation de ces dérivés et l'utilisation de ceux-ci dans le procédé de préparation de dérivés tétrahydrofuranne à groupe triazole. L'invention concerne également les composés nouveaux obtenus lors des différentes étapes du procédé.

Les composés tétrahydrofuranne à groupe triazole sont des produits fongicides connus. Ils sont notamment décrits dans les demandes de brevet européen n° 0121 979 et/ou 0151 084.

Un but de l'invention est de fournir de nouveaux composés permettant, notamment, la réalisation d'un procédé de préparation des tétrahydrofurannes à groupe triazole avec un rendement et des conditions réactionnelles améliorées.

L'invention concerne donc les composés de formule I indiquée dans le tableau annexé à la fin de la description et faisant partie intégrante de celle-ci dans laquelle :

- $R_2$, $R_3$, $R_4$, identiques ou différents, représentent l'atome d'hydrogène, ou un radical alkyle inférieur, cycloalkyle inférieur, aryle (notamment phényle), éventuellement substitué par un ou plusieurs atomes ou radicaux tels que les atomes d'halogène, les radicaux alkoxy inférieur, aryle (notamment phényle), alkyle inférieur, halogénoalkyle inférieur, halogénoalcoxy inférieur, aryloxy (notamment phénoxy), hydroxy
- X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano dans le cas où $R_3$ et/ou $R_4$ correspondent à l'atome d'hydrogène.
- n est un nombre entier, positif ou nul, inférieur à 6, de préférence égal à 2, étant entendu que lorsque n est supérieur à 1, les substituants X peuvent être soit identiques, soit différents,
- m = 0 ou 1,
- Y correspond à un atome ou groupement susceptible d'être éliminé au moyen d'une substitution nucléophile, éventuellement aprés transformation intermédiaire appropriée,

leurs sels acceptable en agriculture.

L'invention concerne donc également les formes salifiées des composés selon l'invention. Les formes salifiées sont les formes acceptables en agriculture parmi lesquelles on peut citer : les chlorhydrate, sulfate, oxalate, nitrate ou arylsulfonate ou aralkylesulfonate ou alkylsulfonate ainsi que les complexes d'addition de ces composés avec des sels métalliques, et notamment des sels de fer, chrome, cuivre, manganèse, zinc, cobalt, étain, magnésium et aluminium.

A titre d'exemple, des complexes avec le zinc peuvent être obtenus en faisant réagir le composé de formule I avec le chlorure de zinc.

Le terme "inférieur" qualifie un radical organique comportant au plus six atomes de carbone. Ce radical peut être linéaire ou ramifié.

Par atome ou groupement susceptible d'être éliminé au moyen d'une substitution nucléophile on entend notamment les atomes d'halogène, de préférence le chlore ou le brome, les groupements hydroxyles ou thiols. Ces derniers toutefois n'étant pas suffisamment mobiles doivent être transformés en groupements partant intermédiaires parmi lesquels on peut citer : le tosylate, le mésylate, le triflate ou le groupement de formule $[Ph_3P^+ - O^-]$.

La transformation en groupe partant s'éffectue de manière connue par action des chlorures d'acide correspondants : chlorure de tosyle, chlorure de mésyle ou par action de l'anhydride trifluorométhanesulfonique en présence d'une amine tertiaire ou de pyridine par exemple, ou en milieu neutre par action du diazodicarboxylate d'éthyle et de la triphénylphosphine.

Le procédé de préparation des composés selon l'invention est caractérisé en ce qu'on isomérise un composé de formule II dans laquelle $R_2$, $R_3$, $R_4$, X, n, m,Y, ont la même signification que dans le composé de formule I, en présence d'un catalyseur, en phase homogène ou hétérogène.

Parmi les catalyseurs utilisables pour assurer l'isomérisation, on citera avantageusement les métaux de transition suivant : ruthenium, cobalt, palladium, nickel, rhodium, iridium, platine, fer.

Ces catalyseurs peuvent fonctionner sous forme hétérogène à l'état métallique, et dans ce cas ils sont déposés sur un support inerte approprié tel que le noir de charbon. De préférence ils peuvent également fonctionner en catalyse homogène sous la forme de complexes comportant outre le métal de transition, un ou plusieurs ligands appropriés (phosphine, carbonyle) et une ou plusieurs molécules d'hydrures.

La réaction peut être effectuée en masse ou en présence d'un solvant protique ou aprotique. Parmi les solvants aprotiques on peut citer les hydrocarbures aliphatiques saturés tels que le n-pentane, l'isopentane, le 2-méthylhexane, le 2,2,5-triméthylhexane, les hydrocarbures aromatiques tels que le benzène, le toluène, l'éthylbenzène, les éthers aliphatiques saturés tels que le tétrahydrofuranne, l'isopentyléther, les éthers aromatiques tels que le benzyléthyléther, les cétones aliphatiques saturées ou aromatiques comme la méthylethylcétone, l'acétophénone, les hydrocarbures halogénés saturés aliphatiques ou aromatiques comme le flucrobenzène, le 1-chloro-2-méthylpropane, le chlorure d'isobutyle, les esters aliphatiques saturés ou aromatiques comme l'isobutyrate d'isobutyle, l'acétate d'éthyle, le benzoate de méthyle. Tous ces solvants

peuvent être présents seuls ou en mélange.

Soulignons que la nomenclature employée est française mis à part le fait que la place des substituants est indiquée en amont des dits substituants.

Parmi les solvants protiques on peut citer les alcools aliphatiques saturés ou aromatiques tels que le méthanol, l'isopropanol, le phénol, les acides aliphatiques saturés ou aromatiques comme l'acide propanoïques, l'acide benzoïque. On peut également utiliser un acide minéral en mélange avec un solvant organique précité dans lequel il est miscible par exemple l'acide chlorhydrique dans le méthanol. Dans ce cas on obtient directement le composé de formule V ci-après décrite dans laquelle $R_1 = CH_3$. Tous ces solvants peuvent se présenter en mélange ou seuls.

Parmi ces solvants on préfère les hydrocarbures aromatiques ou les alcools inférieurs aliphatiques saturés, éventuellement halogénés comme le trifluoroéthanol. Si la réaction s'effectue en présence d'un solvant, la dilution du produit réactionnel sera notamment comprise entre 0,5 % et 99 % en poids par rapport au poids total de la solution et de préférence entre 5 et 50 %.

La quantité de catalyseur mise en jeu sera de préférence comprise entre 0,00005 et 0,1 mole par mole de produit de formule II.

La température d'isomérisation peut varier dans une large gamme par exemple entre -20°C et la température de décomposition du catalyseur. Néanmoins un bon compromis entre la durée de la réaction et des conditions opératoires industrielles conduisent à préférer une gamme de température allant de 0°C à 100°C avantageusement entre 10°C et 80°C.

La pression est généralement comprise entre 1 et 10 atmosphères (soit 0,1 à 1 MPa).

Un procédé avantageux mais non obligatoire pour synthétiser les composés de formule II dans le cas où Y correspond à un atome d'halogène consiste à cycliser le composé de formule VI sous forme cis dans laquelle X, n, m, $R_2$, $R_3$, $R_4$ ont la même signification que pour le composé de formule I et Hal est un atome d'halogène soit en milieu acide si Z est un atome d'hydrogène, soit en milieu basique si Z est un groupe partant tel qu'un mésylate, un tosylate, un triflate, un groupement de formule [$Ph_3P$+ - O -].

Ces groupes partants sont fixés au groupe hydroxyle comme précédemment et de façon sélective sans toucher au groupe hydroxyle tertiaire.

Les catalyseurs acides organiques ou minéraux conviennent pour cette cyclisation. Ceux-ci peuvent être solubles dans le milieu réactionnel ou insolubles, protiques ou aprotiques. Comme acides protiques on peut citer les acides chlorhydrique, sulfurique, trifluoroacétique, perchlorique, benzène-sulfonique, toluène-sulfonique, méthane-sulfonique. Comme acides aprotiques on peut citer les acides de Lewis tels que $BF_3$, $AlCl_3$ et $SnCl_4$.

On utilisera de préférence de 0,1 à 2 équivalent molaire d'acides.

On peut également effectuer cette cyclisation au moyen de catalyseurs fixés sur des supports inertes tels que les résines sulfoniques.

La cyclisation s'effectue ordinairement par simple chauffage des réactifs indiqués. La température est généralement comprise entre 10°C et 100°C ou si il y a un solvant entre 10°C et la température d'ébullition du solvant considéré.

Parmi les nombreux solvants utilisables on peut citer les solvants aliphatiques, aromatiques tel que le toluène, les éthers et les cétones.

Dans le cas de la cyclisation en milieu basique, on peut mentionner à titre indicatif des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. On utilisera de préférence de 0,01 à 2 équivalent molaire de base. On peut également effectuer cette cyclisation au moyen de catalyseurs fixes sur des supports inertes tels que les résines. La réaction s'effectue ordinairement à une température comprise entre 10°C et 100°C et éventuellement en présence d'un solvant tel que les solvants aliphatiques, aromatiques, les éthers, les cétones.

Les composés de formule II dans laquelle Y correspond à un groupement hydroxy peuvent être obtenus par traitement basique des produits de formule générale VI dans laquelle Z = H.

On passe alors par l'intermédiaire des époxydes correspondants issus des produits de formule générale générale VI. Il est par la suite nécessaire après l'isomérisation de transformer le groupe Y = hydroxy en groupe partant (mesylate, tosylate, triflate, $PH_3P$+-O) avant la réaction de subtitution par le triazole comme cela sera décrit ci-après.

Dans le cas où les groupements $R_3$ et $R_4$ correspondent tous les deux à l'atome d'hydrogène, de préférence les composés de formule VI sont obtenus par hydrogénation du composé de formule VII où X, n, m, $R_2$, Y, ont la même signification que dans le composé de formule II et dans laquelle Pr représente un groupe protecteur comme le 1-éthoxy éthyle ou l'atome d'hydrogène, au moyen d'une quantité équimoléculaire d'hydrogène en présence d'un catalyseur approprié éventuellement empoisonné choisi parmi les catalyseurs suivants : palladium, ruthénium, nickel de Raney, platine, rhodium et de préférence le palladium éventuellement empoisonné (pyridine, quinoléine, tétrahydrothiophène) qui donne spécifiquement l'oléfine cis.

Comme précédemment cette réaction peut être effectué en phase homogène ou hétérogène.

De préférence on choisira le palladium à l'état métallique déposé sur un support inerte tel que le noir de carbone, le carbonate de calcium ou le sulfate de baryum.

La réaction bien que cela ne soit pas indispensable peut avantageusement être effectuée en solvant polaire protique par exemple dans un alcool inférieur tel que le méthanol ou aprotique par exemple dans le toluène.

La dilution du composé de formule VII est de préférence comprise entre 1 et 80 % en poids ou mieux 5 % -40 % par rapport à la solution totale.

De même si la proportion molaire de catalyseur par rapport au composé de formule VII peut varier de manière considérable il sera préférable pour des raisons industrielles évidentes d'utiliser le catalyseur dans une proportion molaire comprise entre 0,01 et 0,5 % par rapport au composé de formule VII.

La réaction s'effectue à des températures comprises entre -20°C et +150°C de préférence entre 10 et 80°C et sous pression de 1 à 10 atmosphères (soit 0,1 à 1 MPa).

Le composé de formule VII peut être obtenu par des procédés bien connus de l'homme de l'art par exemple par addition de l'organomagnesien de formule :

$R_2$-(OPr)CH-C≡C-Mg-Br

sur une acétophénone de formule :

(X)n Ph($CH_2$)m $COCH_2$Hal

dans le cas où Pr est un groupe protecteur, il est nécessaire de déprotéger l'alcool avant la cyclisation.

La réaction peut s'effectuer par exemple dans le tétrahydrofuranne de manière connue.

Dans le cas ou au moins un des groupes $R_3$ ou $R_4$ ne correspondent pas à l'atome d'hydrogène, il est possible d'utiliser la voie de synthèse suivante : addition d'un organomagnésien de formule $R_4$MgX sur les composés de formule générale VII, en présence ou non d'iodure de cuivre suivie éventuellement de l'addition d'un halogénure d'alkyle $R_3$X dans un solvant tel que tétrahydrofuranne. Dans le cas où l'on s'arrête à l'addition de $R_4$MgX il est bien évident que l'on fait suivre cette étape d'addition d'une hydrolyse.

L'invention a également pour objet l'utilisation des composés de formule I comme intermédiaires pour la préparation de composés de formule III dans laquelle W représente un groupe trivalent constitué soit d'un groupe =CH- soit d'un atome d'azote =N-,$R_1$ représente l'atome d'hydrogène ou un radical alkyle inférieur, cycloalkyle inférieur, aryle (notamment phényle), aralkyle (notamment benzyle), ces divers radicaux pouvant être éventuellement substitués par un ou plusieurs atomes ou radicaux tels que les atomes d'halogène, les radicaux alkoxy inférieur, aryloxy, hydroxy et $R_2$, $R_3$, $R_4$, X, n, m ont la même signification que pour le composé de formule I, l'utilisation étant caractérisée en ce qu'elle consiste soit dans le greffage d'un noyau imidazole ou triazole sur le composé de formule I de manière à obtenir un composé de formule IV suivie del'addition en milieu acide d'un composé de formule $R_1$OH ou soit de l'addition en milieu acide d'un composé de formule $R_1$OH sur le composé de formule I de manière à obtenir un composé de formule V suivie du greffage d'un noyau imidazole ou triazole

L'étape de greffage est avantageusement effectuée en présence d'un accepteur d'acide, en milieu anhydre ou non anhydre, dans un solvant, inerte dans les conditions de la réaction, généralement entre 50 et 180°C et de préférence à une température voisine de la température d'ébullition du solvant. Comme accepteurs d'acide, on peut mentionner des bases minérales comme par exemple la soude ou la potasse, les carbonates de métaux alcalins ou de métaux alcalinoterreux, des bases azotées comme la triéthylamine, des amines quaternaires comme le tétrabutylammonium hydroxyde, ou des hydroxydes de phosphonium. Comme solvants on utilise avantageusement des solvants aprotiques polaires, tels que par exemple, le diméthylformamide, le diméthylacétamide, le diméthylsulfoxyde, l'acétone, la méthyl-éthyl-cétone, l'acétonitrile, la N-méthylpyrrolidone ou des solvants protiques polaires tels que le méthanol ou le propanol. Si désiré, cette réaction peut être effectuée en présence d'un catalyseur approprié. Comme catalyseur utilisable, on peut mentionner des catalyseurs de transfert de phases, tels que par exemple, des dérivés d'ammonium quaternaires comme le chlorure de tétrabutylammonium.

La réaction est de préférence effectuée en excès molaire de dérivé de triazole ou imidazole de préférence compris entre 1,05 et 1,5.

Lorsque l'on utilise un solvant, on préfèrera travailler dans un milieu dilué comportant 1 % à 70 % en poids de composé de formule I ou V par rapport à la solution totale.

L'accepteur d'acide est au moins présent en quantité stoechiométrique en équivalent d'atome d'hydrogène labile du triazole ou imidazole. En général un rapport en équivalent molaire compris entre 1 et 2,5 sera satisfaisant.

Il est évident que le dérivé salifié du triazole ou imidazole peut être préparé à part et dans ce cas la présence d'un accepteur d'acide n'est pas requise pour la réaction de greffage. Cette préparation s'éffectue en milieu anhydre ou non anhydre dans un solvant dans les mêmes conditions opératoires que celles décrites dans le cas de la formation in situ du dérivé triazole ou imidazole salifié.

En ce qui concerne la réaction d'addition, celle-ci s'effectue en milieu acide.

L'acide catalyseur mis en oeuvre dans cette réaction peut être un acide protique ou aprotique, soluble ou insoluble. Comme acides protiques on peut citer les acides chlorhydrique, sulfurique, trifluoroacétique, perchlorique, benzène-sulfonique, toluène-sulfonique, méthane-sulfonique. Comme acides aprotiques on peut citer les acides de Lewis tels que $BF_3$, $AlCl_3$ et $SnCl_4$. On peut également utiliser les résines solides comme les résines sulfoniques.

On utilisera de préférence de 0,1 à 2 équivalents molaire d'acides par mole de composé de formule II.

La réaction s'effectue ordinairement par simple chauffage des réactifs indiqués. La température est généralement comprise entre 10°C et 100°C ou si il y a un solvant entre 10°C et la température d'ébullition du solvant considéré. Comme solvant on peut notamment utiliser un hydrocarbure aliphatique, alicycLique ou

aromatique, halogéné ou non, un éther, ou un alcool tel que celui de formule $R_1OH$.

De préférence le rapport molaire $R_1OH$ : composé de formule II, sera supérieur à 1 avantageusement entre 1 et 100.

Si l'on utilise un solvant la dilution en poids du composé de formule II par rapport à la solution totale sera avantageusement comprise entre 5 et 70 % en poids.

En fin de réaction, quelque soit le procédé utilisé, le composé III formé est isolé du milieu réactionnel par tout moyen en soi connu tel que, par exemple par distillation du solvant, ou par cristallisation du produit dans le milieu réactionnel, ou par filtration, puis si nécessaire, ce composé est purifié par des méthodes usuelles telles que recristallisation dans un solvant approprié.

Il est également posible de greffer un noyau imidazole ou triazole sur le composé de formule II selon l'étape de greffage décrite précédemment puis ensuite d'isomériser la double liaison en présence d'un catalyseur, en phase homogène ou hétérogène comme c'est décrit précédemment.On passe dans ce cas par un composé de formule VIII.

L'invention a également pour objet les composés de formule II, III, IV, VI, VII dans lesquelles X, $R_1$ à $R_4$, m, n, Y, Hal, ont la même signification que dans le composé de formule I.

l'invention a également pour objet l'utilisation des composés de formule IV et de ses sels acceptables en agriculture précédemment définis à titre d'herbicide. Ces composés revèlent en effet d'excellentes propriétés notamment contre les oidium, piétin verse et en général les autres maladies fongiques des céréales.

L'invention a également pour objet un procédé de préparation d'éther de propargyle de formule (I)
$R_1O-CHR_2-C\equiv CH$
dans laquelle $R_1$ et $R_2$ ont la même signification que précédemment.

Il est connu dans l'art d'obtenir des éthers de formule $XCH_2-O-CRR'-C\equiv CH$
où X est $CH_2OH$, $CF_3$ ou $-C\equiv CH$ et R, R' sont H ou alkyle inférieur par action du bromure de formule $BrCH_2-C\equiv CH$ sur l'éthanolate de sodium correspondant en présence d'un solvant aprotique polaire à température ambiante. Ce procédé est décrit dans le brevet US 3 077 501.

Toutefois, ce procédé présente l'inconvénient de faire intervenir le bromure de propargyle, produit instable difficilement utilisable industriellement.

Par ailleurs, le procédé de l'art antérieur quand on remplace le bromure par le chlorure ne conduit pas à l'éther de propargyle attendu.

Ainsi un objet de la présente invention est de proposer un procédé industriel permettant d'obtenir l'éther de propargyle de formule (I).

Un autre objet de l'invention est de proposer un procédé de préparation d'éther de propargyle avec un rendement et des conditions opératoires améliorées.

Selon l'invention le procédé consiste à mettre en contact un composé de formule (II) $R_1OM$ (M métal alcalin) avec un chlorure de propargyle de formule (III)

$$Cl-\underset{\substack{|\\ R_2}}{CH}-C\equiv CH \qquad III$$

dans laquelle $R_2$ a la même signification que dans la formule (I), en présence d'un halogène alcalin.

Dans la présente description le terme "inférieur" qualifie un radical organique comportant au plus 6 atomes de carbone. Ce radical peut être linéaire ou ramifié.

Ce procédé est particulièrement intéressant dans le cas où $R_1$ est un radical substitué par un ou plusieurs atomes d'halogène notamment $-CH_2CF_3$, $-CH_2CH_2Cl$, $-CH_2CH_2F$, $CH_2OCH_3$, $CH_2OC_2H_5$.

De plus, selon une autre variante préférée prise ou non en combinaison avec la précédente $R_1$ est un radical alkyle $C_1$- $C_4$.

Parmi les halogénures alcalins, on préférera les iodures alcalins, de préférence l'iodure de sodium ou de potassium.

De préférence la réaction est effectuée en présence d'un solvant qui est avantageusement un solvant aprotique polaire tel que par exemple, le diméthyl formamide, le diméthyl acétamide, le diméthyl sulfoxyde, l'acétone, la méthyl éthyl cétone, la méthyl isolutyl cétone, l'acétonitrile, la N-méthyl pyrrolidone. Si nécessaire, on peut utiliser un mélange liphasique eau + solvant précédemment cité en présence éventuellement d'un catalyseur de transfert de phase comme un dérivé d'ammonium quaternaire (par exemple le chlorure de tétrabutylammonium.

La température de la réaction est avantageusement comprise entre la température ambiante et le reflux du solvant s'il y en a un.

Lorsqu'on utilise un solvant, on préférera travailler dans un milieu dilué comportant 1 % à 70 % en poids de composé de formule (II) et (III), par rapport à la solution totale.

De préférence, le rapport molaire II : III est compris entre 0,5 et 2 avantageusement voisin de 1.

La quantité molaire d'halogénure alcalin peut être très inférieure à celle du composé de formule (II).

Elle est avantageusement 2 à 100 fois inférieure.

Exemple 1 : Préparation du 2-chlorométhyl 2-(2,4-dichloro 1-phényl) 2,3-dihydrofuranne

Dans un ballon de 25 ml sous atmosphère inerte, on introduit une solution de 2-chlorométhyl 2-(2,4-dichloro 1-phényl) 2 5-dihydrofuranne (3,95 g, 15 mmoles) dans 7,5 ml de méthanol. On ajoute 144 mg (0,15 mmoles) de $RuH_2(PPH_3)_4$ et on porte au reflux. La réaction est terminée après 12 h. On élimine le méthanol sous pression réduite. Après purification on obtient une huile incolore (3,817 g, 14,48 mmoles) - rendement 96,5 % par rapport au produit de départ.

Préparation du 2,5 dihydrofuranne 2-chlorométhyl 2-(2,4-dichloro 1-phényl)

Dans un ballon de 100 ml sous atmosphère inerte, on introduit 10 g (35,5 mmoles) de 1-chloro 2-(2,4-dichloro 1-phényl) 3-pentène 2,5-diol (isomère cis) dans 60 ml de toluène. On ajoute 0,5 g d'acide paratoluène sulfonique et on porte au reflux. La réaction terminée on lave et sépare. On obtient un résidu huileux brun 9,36 g (35,5 mmoles) -Rendement 100 % par rapport au produit de départ.

Préparation du 1-chloro 2-(2,4 dichloro 1-phényl) 3-pentène 2,5 -diol (isomère cis)

Méthode A :

Dans un ballon de 500 ml sous atmosphère inerte et contenant 13,37 g de magnésium et 30 ml de tétrahydrofuranne on coule en 2h30 54,5 g (0,5 moles) de bromoéthane en solution dans 225 ml de THF à T = 30° C. On coule la solution obtenue goutte à goutte en 1 heure à température ambiante sur une solution d'éther de 2-éthoxyéthyle et de propargyle (64,09 g ; 0,5 mole) dans le THF (40 ml). Dans la solution précédente on coule à 0° C en 2 heures une solution de 2,4,2'-acétophénone (89,4 g ; 0,4 moles) dans 100 ml de THF. On maintient 6 h à 20° C. On refroidit à 0° C et on ajoute en 15 mn à 5° C de l'acide acétique (28,6 ml ; 0,5 mole) puis 150 ml d'eau puis 100 ml d'éther éthylique. La phase organique est lavée avec 2 fois 50 ml d'eau et une fois avec 50 ml de saumure puis les solvants sont éliminés sous pression réduite. On obtient 136,6 g d'une huile jaune visqueuse. Rendement : 97,2 % par rapport au produit de départ.

Le produit est identifié comme le 1-chloro 2-(2,4-dichloro 1-phényl) 5-[(1-éthoxyéthoxy)] 3-pentyne 2-ol.

Dans un ballon de 500 ml sous atmosphère inerte on introduit 24 g de ce dernier en solution dans le toluène (150 ml), 0,613 g de palladium à 5 % sur charbon. On alimente en hydrogène sous pression atmosphérique à 25°C. Au bout de 2 heures, on filtre puis le solvant est éliminé sous pression réduite.

Le résidu huileux est repris au méthanol (200 ml) et on ajoute de l'acide chlorhydrique 0,5 N (50 ml). Le méthanol est alors éliminé sous pression réduite et on obtient 19,36 g d'un résidu huileux orange.

L'addition de 10 ml d'acétate d'éthyle puis de 35ml de pentane permet la précipitation de 5,35 g (19,3mmoles) de diol souhaité.

Méthode B :

Dans un ballon de 1 l sous atmosphère inerte et contenant du magnésium (36,5 g ; 1,5 moles) et THF (216 g) et du toluène 75 ml en solution, on coule à 30° C en 1h15, 163,45 g de bromoéthane dans 250 ml de toluène. On laisse reposer 15 mn à 24° C. On coule goutte à goutte une solution d'alcool propargylique (42,15 g ; 0,75 mole) dans le toluène (50 ml) en 1h30. On ajoute goutte à goutte à 44° C une solution de 2,4,2'-trichloroacétophénone (111,7 g ; 0,5 mole) dans le toluène (100 ml). On laisse reposer à température ambiante. Le milieu est alors refroidi à 0° C et on ajoute goutte à goutte 90 g (1,5 mole) d'acide acétique. On évapore, ajoute du toluène (250 ml) et lave à l'acide sulfurique dilué puis à l'eau. La phase organique est alors concentrée, refroidie, précipitée, filtrée, séchée. On obtient le 1-chloro 2-(2,4-dichloro 1-phényl) 3-pentyne 2,5 diol. P.F = 90° C. L'hydrogénation est effectuée de la même manière que pour la méthode A mais à 50° C. On obtient le diol souhaité.

Exemple 2 Préparation du 2-(2,4-dichloro 1-phényl) 2-méthyl (1-triazol) 5-trifluoroethoxy tétrahydrofuranne.

Préparation du 2-(2,4-dichloro 1-phényl) 2-méthyl (1-triazolyl) 2,3-dihydrofuranne :

Dans un ballon de 50 ml sous atmosphère inerte, on introduit une solution de composé préparé selon l'exemple 1 (1,000 g ; 3,80 mmoles) dans la N-méthyl pyrrolidone (N.M.P.) (1,00 g). On ajoute alors le triazole -1,2,4 (314,8 mg ; 4,56 mmoles) et du carbonate de potassium (630,2 mg ; 4,56 mmoles). Le milieu réactionnel est porté à une température de 170° C pendant 14 heures puis est refroidi au voisinage de 20° C. On ajoute alors 10 ml de toluène puis de l'eau saturée de chlorure d'ammonium. La phase toluénique est recueillie, la phase aqueuse est extraite avec encore 2 fois 10 ml de toluène. Les extraits organiques réunis sont séchés sur sulfate de sodium. Après filtration et concentration sous pression réduite, on obtient un résidu semi cristallin qui est purifié. Poids obtenu : 673 mg (2,28 mmoles) ; PF (Kofler) : 100° C. Rendement 60 % par rapport au dihydrofuranne-2,3 de départ.

Préparation de 2-chlorométhyl 2-(2,4-dichloro 1-phényl) 5-trifluoroethoxy tétrahydrofuranne

Dans un ballon de 10 ml sous atmosphère inerte, on introduit 0,528 g ( 2 mmoles) de 2-(2,4-dichloro 1-phényl) 2-chloro méthyl) 2,3 dihydrofuranne, du trifluoroéthanol (1 ml ; 13 mmoles), du chlorure de méthylène (0,5 ml) et quelques grains d'acide paratoluène sulfonique. Ce mélange est porté à une température comprise entre 40 et 50° C pendant une heure ; temps au bout duquel la réaction est totale. Les solvants sont alors éliminés sous pression réduite (30 mm de mercure - 40° C) on obtient un mélange 60/40 de deux

diasterioisomères (0,713 g ; 1,96 mmoles). Rendement 98 % par rapport au produit de départ.

Préparation de 2-(2,4-dichloro 1-phényl)2-méthyl (1-triazolyl) 5-trifluoroéthoxy tétrahydrofuranne

Méthode A

Dans un ballon de 10 ml sous atmosphère inerte, on introduit 600 mg ( 1,65 mmoles) de tétrahydrofuranne préparé précédemment et 1,7 ml de N-méthylpyrrolidone (NMP). On ajoute alors du traizolate de sodium (0,180 g ; 1,98 mmoles). Le mélange est porté à 160° C. Au bout de 5 heures le rendement est de 15 % en chromatographie liquide.

Méthode B

Dans un ballon de 25 ml, on introduit 0,6 g ( 2 mmoles) de 2-(2,4 dichloro 1-phényl) 2-méthyl (1-triazolyl) 2,3 dihydrofuranne en solution toluènique, 1 ml (13,7 mmoles) de trifluoroéthanol. Le milieu est alors porté au reflux et traversé par un courant d'Hcl gazeux sec. Après une heure de chauffage la réaction est terminée. Le milieu est alors repris au chlorure de méthylène, lavé avec une solution saturée de carbonate de sodium puis à l'eau et séché sur sulfate de sodium. Après filtration, concentration, purification, on récupère 0,440 g (1,1 mmole) d'un solide blanc fondant à 168° C. Rendement 80,7 % par rapport au produit de départ.

Préparation du 2-(2,4-dichloro 1-phényl) 2-méthyl (1-triazolyl) 2,5-dihydrofuranne :

Dans un ballon de 50 ml sous atmosphère inerte, on introduit une solution de 2,5 dihydrofuranne 2-chlorométhyl 2-(2,4-dichloro 1-phényl)(1,000 g ; 3,80 mmoles) dans la N-méthyl pyrrolidone (N.M.P.) (1,00 g). On ajoute alors le triazole -1,2,4 (314,8 mg ; 4,56 mmoles) et du carbonate de potassium (630,2 mg ; 4,56 mmoles). Le milieu réactionnel est porté à une température de 170° C pendant 14 heures puis est refroidi au voisinage de 20° C. On ajoute alors 10 ml de toluène puis de l'eau saturée de chlorure d'ammonium. La phase toluénique est recueillie, la phase aqueuse est extraite avec encore 2 fois 10 ml de toluène. Les extraits organiques réunis sont séchés sur sulfate de sodium. Après filtration et concentration sous pression réduite, on obtient un résidu semi cristallin qui est purifié.

Ce produit est ensuite isomérisé selon les conditions de l'exemple 1 pour obtenir un composé de formule IV sur lequel on additionne ensuite le trifluoroéthanol.

Exemple 3 : Préparation du ditrifluoroéthoxy-2,3 propène

Au sel de sodium de trifluoroéthano -préparé par réaction de 4,8 g (0,2 mole) de NaH sur 20 g (0,2 Mole) d'alcool- en solution dans 200 ml de THF, sont additionnés 0,05 g de NaI et 14,9 g (0,2 Mole) de chlorure propargylique.

Après 24 heures d'agitation au reflux du solvant, le milieu réactionnel est filtré, le précipité est lavé avec 40 ml de THF puis le filtrat est distillé.

On récupère ainsi 11,44 g (0,063 mole) de composé passant entre 70 et 80° C sous 96 mm de mercure (Rendement/TFE = 63 %).

Exemple 4 : Préparation du trifluorométhoxy-3 propyne I

Dans un tétracol de 250 ml muni d'une agitation mécanique, d'un thermomètre, d'une ampoule de coulée et d'un réfrigérant, on introduit sous agitation et sous atmosphère inerte 150 ml de N,N-diméthylformamide sec, 60.02 g (0,6 mole) de trifluoroéthanol, 82,9 g (0,6 mole) de carbonate de potassium et 4,49 g (0,03 mole) d'iodure de sodium.

A la suspension obtenue, on coule 49,17 g (0,66 mole) de chlorure propargylique. La masse réactionnelle est alors chauffée pendant 18 heures à 85° C.

Après refroidissement, le milieu est filtré et le précipité obtenu lavé avec 40 ml de DMF. Le filtrat est alors distillé pour conduire à 52,2 g (0,38 mole) du composé ; liquide incolore, $pE_b$ = 81-82° C/P.atm.). Rendement = 63 %.

Exemple 5 : Préparations du chloro-5 (dichloro-2', 4' phényl)-4 hydroxy-4 trifuloroéthoxy-1 pentyne-2

Le réactif de grignard du bromoéthane est préparé de façon classique à partir de 6,74 g (0,27 mole) de magnésium, 27,46 g (0,25 mole) d'halogénure et de 180 ml de THF anhydre. Ce réactif, filtré pour éliminer l'excès du magnésium, est ensuite coulé lentement sur une solution de 34,8 g (0,252 mole) de composé de l'exemple 4 dans 60 ml de THF anhydre. La réaction exothermique provoque un dégagement d'éthane qui cesse peu après la fin de la coulée.

Une solution de 31,3 g (0,14 mole) de trichloroacétophénone dans 35 ml de THF anhydre est alors additionnée lentement (réaction exothermique) au milieu réactionnel.

A la fin de l'addition et après refroidissement, le milieu est coulé sur une solution glacée de 21 ml d'HCL 12 N dans 100 ml d'$H_2O$.

Après décantation, extraction avec 2 X 100 ml d'$Et_2O$, lavage à neutralité, sèchage au sulfate de sodium et concentration, on récupère 49 g d'un liquide orangé qui dose 93 % (rendement = 90 %).

Le brut réactionnel est utilisé tel quel dans la phase suivante.

Exemple 6 : Préparation du chloro-5 (dichloro-2', 4' phényl)-4 hydroxy-4 trifluoroéthoxy-1 pentène-2

Une solution de 34 g (0,094 mole) d'acétylénique dans 120 ml de toluène est hydrogénée en présence de 1 g de palladium sur charbon à 5 % sous pression atmosphérique et à 65°C.

Après consommation d'un équivalent d'hydrogène, l'agitation est arrêtée. Après refroidissement, le milieu est filtré et le filtrat concentré. On obtient ainsi 34,2 g d'un brut réactionnel estimé à 90 % en composé éthylénique (rendement = 90 %).

Le composé éthylénique est obtenu très majoritairement sous sa configuration CIS. Il peut être isolé du brut réactionnel par chromatographie sur colonne de silice (Eluant : Pentane/$CH_2Cl_2$ = 60/40).

Exemple 7 : Préparation du chloro-5 (dichloro-2', 4' phényl)-4 hydroxy-4 trifluoroéthoxy-1 pentène .

Une solution de 2,24 g ($6,16.10^{-3}$ moles) de dérivé éthylénique dans 2,2 ml de toluène est dportée au reflux en présence de 0,07 g ($6,10^{-5}$ moles) de $RuH_2$ $(PPh_3)_4$. Après une heure de réaction, on laisse revenir à température ambiante. Le milieu est dilué avec 5ml de toluène et filtré sur 5 g de silice. Après concentration, on récupère 1,8 g ($4,9\ 10^{-3}$ moles) d'un mélange CIS (58 %) et TRANS (42 %) de l'éther vinylique . Rendement = 80 %.

Exemple 8 : Préparation du (dichloro-2', 4', phényl)-4 hydroxy-4 triazolyl-5 trifluoro éthoxy-1 pentène

Une suspension de 6,54 g (0,018 mole) d'éther précédent, de 1,35 g (0,019 mole) de triazole et de 5,39 g (0,039 mole) de carbonate de potassium dans 25 ml de DMF sec est chauffée pendant 2 heures à 110°C.

Le milieu est ramené à température ambiante puis dilué avec 40 ml de toluène et 40 ml d'$H_2O$. Après décantation, lavage à neutralité, sèchage et concentration on obtient 7,1 g de brut réactionnel titrant à 85 % en éther d'énol triazolé .

Après purification par chromatographie sur colonne de silice (éluant : AcOEt/$CH_2Cl$ = 50/60) on obtient 4,98 g (0,0126 mole) de l'éther d'énol (CIS/TRANS = 56,44) (Rendement en produit isolé = 70 %).

Exemple 9 : Préparation du (dichloro-2', 4' phényl)-2 triazolylméthyl-2 trifluoroéthoxy-5 tétrahydrofuranne.

Une solution de 0,476 g ($1,2.10^{-3}$ mole) de composé V dans 3 ml de toluène et 0,722 g ($7,2.10^{-3}$ moles) de trifluoroéthanol est agitée en présence d'un très faible courant d'HCl gaz (réaction exothermique) pendant 2 heures.

Après traitement au carbonate de sodium, lavage à neutralité, sèchage et concentration, on récupère 0,485 g de brut réactionnel contenant 0,404 g ($1,02.10^{-3}$ mole) de composé A (rendement = 85 %).

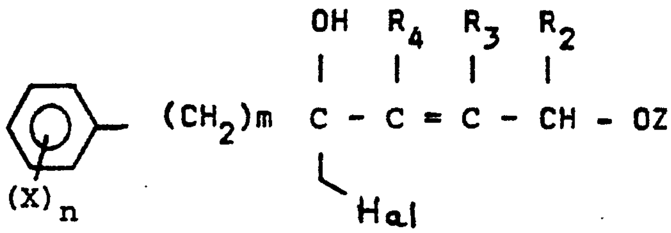

(VI)

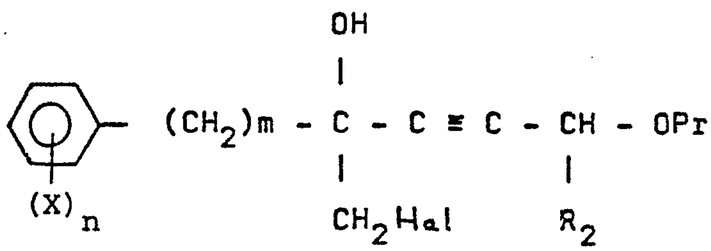

(VII)

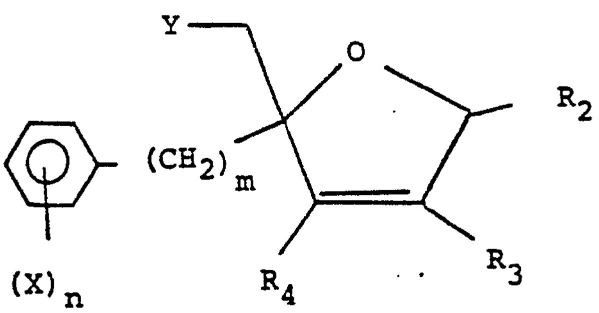

(II)

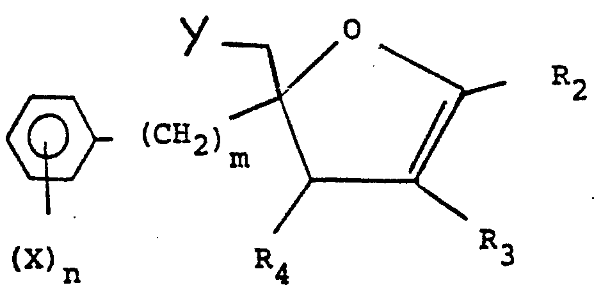

(I)

Tr = noyau 1,2,4-triazole 1-yl ( W = - N=)

Im = noyau 1,3 imidazole 1-yl ( W = -CH=)

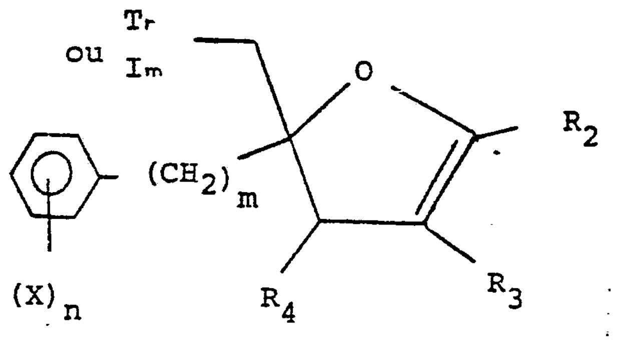

(IV)

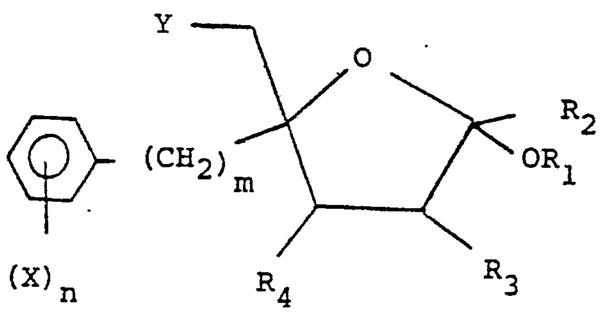

(V)

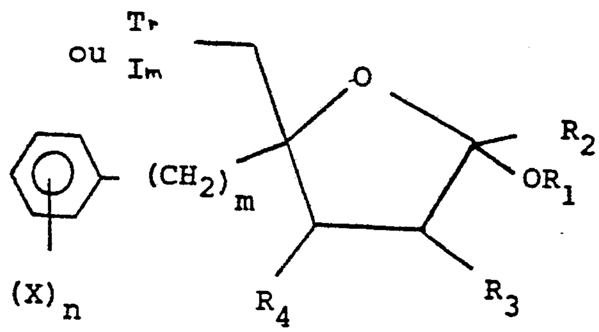

(III)

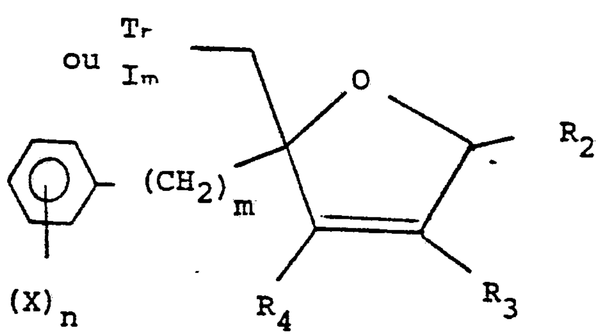

(VIII)

**Revendications**

1 Composés de formule I dans laquelle : $R_2$, $R_3$, $R_4$, identiques ou différents, représentent l'atome d'hydrogène, ou un radical alkyle inférieur, cycloalkyle inférieur, aryle, éventuellement substitué par un ou plusieurs atomes ou radicaux tels que les atomes d'halogènes, les radicaux alkoxy inférieurs, aryle, alkyle inférieur, halogénoalkyle inférieur, halogénoalcoxy inférieur, aryloxy, hydroxy,
X est un atome d'halogène, de préférence le fluor, le brome ou le chlore, ou un groupe alkyle ou alkoxy ayant de 1 à 12 atomes de carbone, de préférence de 1 à 4 atomes de carbone, et étant éventuellement mono ou polyhalogéné (groupement $CF_3$ notamment), ou un groupe cyano dans le cas où $R_3$ et/ou $R_4$ correspondent à l'atome d'hydrogène.
n est un nombre entier, positif ou nul, inférieur à 6, de préférence égal à 2, étant entendu que lorsque n est supérieur à 1, les substituants X peuvent être soit identiques, soit différents,
m = 0 ou 1,
Y correspond à un atome ou groupement susceptible d'être éliminé au moyen d'une substitution nucléophile, éventuellement après transformation intermédiaire appropriée leurs sels acceptables en agriculture.

2 Procédé de préparation de composés selon la revendication 1 caractérisé en ce qu'on isomérise un composé de formule II dans laquelle $R_2$, $R_3$, $R_4$, X, Y, n, m ont la même signification que dans la revendication 1 en présence d'un catalyseur, en phase homogène ou hétérogène.

3 Procédé selon la revendication 2, caractérisé en ce que le catalyseur d'isomérisation est choisi dans le groupe formé par : le ruthénium, le cobalt, le palladium, le nickel, le rhodium, l'iridium, le platine, le fer.

4 Procédé selon la revendication 2, caractérisé en ce que la proportion de catalyseur en mole par rapport au composé de formule II est comprise entre 0,0005 et 0,1.

5 Procédé selon la revendication 2, caractérisé en ce que la réaction d'isomération s'effectue en présence d'un solvant et en ce que, de préférence, la quantité de composé de formule II par rapport au poids total de solution est comprise entre 5 et 50% en poids.

6 Procédé selon la revendication 2, caractérisé en ce que la température de la réaction d'isomération est comprise entre 10° C et 80° C.

7 Utilisation des composés selon la revendication 1 comme intermédiaires pour la préparation de composés de formule III dans laquelle W représente un groupe trivalent constitué soit d'un groupe =CH- soit d'un atome d'azote =N-.$R_1$ représente l'atome d'hydrogène ou un radical alkyle inférieur, cycloalkyle inférieur, aryle (notamment phényle), aralkyle (notamment benzyle), ces divers radicaux pouvant être éventuellement substitués par un ou plusieurs atomes ou radicaux tels que les atomes d'halogène, les radicaux alkoxy inférieur, aryloxy, hydroxy et $R_2$, $R_3$, $R_4$, X, n, m ont la même signification que dans la revendication 1 caractérisée en ce qu'elle consiste soit dans le greffage sur le composé de formule I d'un noyau imidazole ou triazole de manière à obtenir un composé de formule IV suivie de l'addition en milieu acide d'un composé de formule $R_1OH$ de manière à obtenir un composé de formule III ou soit dans l'addition en milieu acide d'un composé de formule $R_1OH$ sur le composé de formule I de manière à obtenir un composé de formule V suivie du greffage d'un noyau imidazole ou triazole de manière à obtenir le composé de formule III.

8 Utilisation selon la revendication 7, caractérisée en ce que l'étape de greffage est effectuée au moyen d'un dérivé salifié, d'un imidazole ou triazole formé ou non in situ, et en ce que le rapport molaire dudit dérivé par rapport au composé de formule I ou V est de préférence compris entre 1,05 et 1,5.

9 Utilisation selon l'une des revendications 7 ou 8, caractérisée en ce que la réaction de l'étape de greffage est effectuée en présence d'un solvant et en ce que la quantité de composé de formule I ou V par rapport au poids total de solution est comprise entre 1 et 70 % en poids.

10 Utilisation selon l'une des revendications 7, 8, 9, caractérisée en ce que la témpérature de la réaction de l'étape de greffage est voisine de la température d'ébullition du solvant.

11 Utilisation selon la revendication 7, caractérisée en ce que la réaction d'addition est effectuée en présence de 0,1 à 2 équivalents d'acides par mole de composé de formule I ou IV.

12 Utilisation selon les revendications 7 ou 11, caractérisée en ce que on utilise 1 à 100 mole de composé de formule $R_1OH$ pour 1 mole de composé de formule I ou IV

13 Utilisation selon la revendication 7, caractérisée en ce que la température de réaction d'addition est comprise entre 10° C et 100° C

14 Procédé de préparation des composés de formule II dans laquelle Y est un atome d'halogène et $R_2$, $R_3$, $R_4$, X, m, n, ont la même signification que dans la revendication 1, caractérisé en ce qu'il consiste à cycliser le composé de formule VI soit en milieu acide si Z est un atome d'hydrogène, soit en milieu basique si Z est un groupe partant.

15 Procédé selon la revendication 14, caractérisé en ce que la réaction de cyclisation est effectuée en présence de 0,1 à 2 équivalent molaire d'acide par mole de composé de formule VI.

16 Procédé selon la revendication 14, caractérisé en ce que la réaction de cyclisation est effectuée en

présence de 0,01 à 2 équivalent molaire de base par mole de composé de formule VI.

17 Procédé selon la revendication 14, caractérisé en ce que la température de la réaction est comprise entre 10° C et 100° C ou s'il y a un solvant entre 10° C et la température d'ébullition du solvant considéré.

18 Procédé de préparation des composés de formule II dans laquelle Y est un groupe hydroxy ou un groupe partant OZ, OZ étant de préférence le tosylate, le mésylate, le triflate ou le groupement de formule [$PH_3P^+$-O-] caractérisé en ce qu'il consiste à traiter par une base le composé de formule VI dans laquelle Y est un atome d'halogène, Z est un atome d'hydrogène, et $R_2$, $R_3$, $R_4$, X, m, n, ont la même signification que dans la revendication 1, puis en ce que on transforme éventuellement le groupe Y = OH en groupe partant OZ.

19 Procédé de préparation du composé de formule VI lorsque $R_3$ et $R_4$ correspondent à l'atome d'hydrogène caractérisé en ce que l'on hydrogène par catalyse homogène ou hétérogène un composé de formule VII dans laquelle Hal est un atome d'halogène, $R_1$, $R_2$, m, n, Z ont la même signification que dans la revendication 1.

20 Procédé selon la revendication 19, caractérisé en ce que l'hydrogénation est effectuée par un métal choisi par le groupe suivant : palladium, ruthénium, nickel de Raney, platine, rhodium, déposé sur un support inerte.

21 Procédé de préparation du composé de formule VII dans le cas où au moins l'un des groupes $R_3$ ou $R_4$ ne correspondent pas à l'atome d'hydrogène caractérisé en ce que l'on additionne un organomagnesien de formule $R_4MgX$ sur les composés de formule VII puis que éventuellement on additionne un halogenure d'alkyl de formule $R_3X$.

22 Composés de formules II, IV, VI, VII dans lesquelles X, $R_1$ à $R_4$, m, n, Y, Hal ont la même signification que dans le composé de formule (I).